# EUROPÄISCHE PATENTSCHRIFT

(11) **EP 0 379 551 B1**
(45) Veröffentlichungstag und Bekanntmachung des Hinweises auf die Patenterteilung: **16.02.1994**
(21) Anmeldenummer: 89907743.2
(22) Anmeldetag: 11.07.1989
(51) Int. Cl.: A61F 5/02, A61B 17/56

(54) **KORREKTUR- UND HALTEVORRICHTUNG, INSBESONDERE FÜR DIE WIRBELSÄULE**
CORRECTION AND RESTRAINING DEVICE, IN PARTICULAR FOR THE VERTEBRAL COLUMN
DISPOSITIF DE CORRECTION ET DE MAINTIEN, NOTAMMENT DE LA COLONNE VERTEBRALE

(30) Priorität: 13.07.1988 DE 3823737
(43) Veröffentlichungstag der Anmeldung: 01.08.1990
(73) Patentinhaber: Harms, Jürgen, Prof. Dr., 76337 Waldbronn (DE); Biedermann, Lutz, 78048 Villingen-Schwenningen (DE)
(72) Erfinder: Harms, Jürgen, Prof. Dr., 76337 Waldbronn (DE); Biedermann, Lutz, 78048 Villingen-Schwenningen (DE)
(74) Vertreter: Prüfer, Lutz H., Dipl.-Phys.
(86) Internationale Anmeldenummer: EP8900802
(87) Internationale Veröffentlichungsnummer: WO9000377

(56) Entgegenhaltungen:
- EP-A- 0 242 708
- DE-A- 3 639 810
- DE-C- 3 711 013
- US-A- 4 289 123

## Beschreibung

Die Erfindung betrifft eine Korrektur- und Haltevorrichtung, wie sie insbesondere für die Wirbelsäule verwendet wird.

Aus UNFALLCHIRURGIE, Heft 12 (1986), Seiten 68 - 79 ist das mechanische Prinzip zur dorsalen Stabilisierung der Brust- und Lendenwirbelsäule mit Hilfe einer Korrektur- und Haltevorrichtung bekannt. Aus einem Prospekt "Fixateur Interne für die Wirbelsäule, Original-Instrumente und -Implantate der Schweizerischen Arbeitsgemeinschaft für Osteosynthesefragen-AO" ist ein Fixateur Interne bekannt. Dieser umfaßt Zwei Paare von sogenannten Schanz'schen Schrauben. Von diesen wird ein erstes Paar durch die beidseitigen Bogenwurzeln in den ersten Wirbelkörper und ein zweites Paar durch die beidseitigen Bogenwurzeln in einen zweiten Wirbelkörper eingeschraubt. Die Schanz'schen Schrauben stehen mit einem ungefähr 10 cm langen Schaft dann aus den Wirbelkörpern hervor. Nach dem Einbringen der vier Schanz-Schrauben wird mittels der langen, vorstehenden Schraubenenden bereits manuell eine partielle Reposition der Wirbel möglich. Anschließend werden Gewindestäbe auf jeweils zwei auf der gleichen Seite der übereinanderliegenden Wirbel befindlichen Schanz-Schrauben aufgesteckt. Dann erfolgt erneut eine Einwirkung auf die hervorstehenden freien Enden der Schanz'schen Schrauben und ein Arretieren der Gewindestäbe. Abschließend werden die über die Gewindestäbe überstehenden Teile der Schanz-Schrauben abgeschnitten. Dann wird die Muskulatur und Haut über den Fixateur gelegt, und die Wunde wird geschlossen. Stellt der Operateur am nächsten Tag fest, daß eine Veränderung der Winkelstellung der Wirbel erforderlich wäre, ist dies nicht mehr möglich, da die zur Ausrichtung der Winkelstellung erforderlichen überstehenden Teil der Schanz-Schrauben bereits entfernt sind. Ferner ist die Rotationsfertigkeit der Vorrichtung gering.

Aus der DE 36 39 810 A1 ist ein Implantat zur Wirbelsäulenkorrektur und/oder Stabilisierung bekannt. Das Implantat weist Schrauben mit einem Gewindeschaftteil und einem diesem abgewandten und mit dem Gewindeschaftteil starr verbundenen Aufnahmeteil auf. Jeweils ein Schraubenpaar ist an seinem einen Ende über das jeweilige Aufnahmeteil mit einer Spannstange starr verbunden. Das Schraubenpaar ist ferner an einer Stelle der Schrauben, die einen Abstand von der Verbindung zwischen Aufnahmeteil und Spannstange hat, zusätzlich mit einem Spannstab verbunden. Damit die axiale Richtung der Schrauben und damit der diese aufnehmenden Wirbelkörper von der Parallelstellung abweichend ausgerichtet werden kann, wird die Verbindung zwischen Spannstab und den beiden Schrauben so verstellt, daß auf die Spannstange oder den Spannstab eine Biegeverformung ausgeübt wird. Damit erfolgt eine unkontrollierbare Materialbeanspruchung, die im Laufe der Zeit zu einer Zerstörung der Spannstange und/oder des Spannstabes führen kann, was im Körperinneren natürlich zu nicht vorhersehbaren Problemen führen kann.

Aufgabe der Erfindung ist es, eine Korrektur- und Haltevorrichtung zu schaffen, mit der die Position von Wirbelkörpern im Sinne der Längenverschiebung als auch der Winkelstellung (also Beugen der Wirbelsäule nach vorne oder nach hinten) ohne Biegebeanspruchung dabei zu verwendender Stäbe einstellbar und fixierbar ist. Dieses Einstellen und Fixieren soll besonders einfach möglich sein. Ein Nachjustieren zu einem nach der Operation liegenden Zeitpunkt soll möglich sein. Nach einer Weiterbildung soll ferner eine hohe Festigkeit im Hinblick auf die Wirbelkörper ausgeübte Drehbewegung als auch im Hinblick auf axiale Belastung erreicht werden. Diese Ziele sollen erreichbar sein bei geringer Bautiefe der Vorrichtung.

Diese Aufgabe wird durch eine Korrektur- und Haltevorrichtung nach Anspruch 1 gelöst. Weiterbildungen der Erfindung sind in den Unteransprüchen gekennzeichnet.

Die dabei zu verwendenden Schrauben entsprechen den in der EP-0 242 708 beschriebenen Schrauben. Ähnliche Schrauben sind aus der DE-C-3 711 013 bekannt.

Weitere Merkmale und Zweckmäßigkeiten der Erfindung ergeben sich aus der Beschreibung eines Ausführungsbeispieles anhand der Figuren. Von den Figuren zeigen:
- Fig. 1: eine Seitenansicht der Korrektur- und Haltevorrichtung, teilweise in geschnittener Darstellung;
- Fig. 2: eine Draufsicht auf die in Fig. 1 gezeigte Vorrichtung und
- Fig. 3: einen Schnitt entlang der Linie III-III in Fig. 1.

Die Korrektur- und Haltevorrichtung umfaßt vier Schrauben 1-4 als Knochenschrauben. Das erste Paar Schrauben 1,3 dient beim Einsetzen der Korrektur- und Haltevorrichtung zur Stabilisierung der Wirbelsäule zum Einschrauben in die Bogenwurzeln in einen Wirbelkörper 5, der schematisch angedeutet ist. Das zweite Paar Schrauben 2,4 dient entsprechend zum Einschrauben in die Bogenwurzeln eines zweiten Wirbelköpers 6, der ebenfalls schematisch angedeutet ist.

Jede der Schrauben 1-4 umfaßt einen Gewindeschaftteil 7 mit einem Gewindeteil 9 und einem am kopfseitigen Ende des Gewindeteiles vorgesehenen kugelsegmentförmigen Kopf 10 sowie einen Aufnahmeteil 8. Der Kopf weist auf seiner dem Gewindeteil 9 abgewandten Seite eine sich senkrecht zur Achse des Gewindeteiles erstreckende ebene Fläche 11 auf. Koaxial zu dem Gewindeteil weist die ebene Fläche einen Schlitz oder eine andere Eingriffsmöglichkeit für einen Schraubendreher zum Einschrauben der Schraube in einen Wirbelkörper auf.

Das Aufnahmeteil 8 umfaßt zwei Kopfhälften 12,13 sowie eigen diese zusammenhaltenden Haltering 14. Jede Kopfhälfte weist auf ihrer der anderen Kopfhälfte zugewandten Innenseite einen hohlkugelsegmentförmigen Abschnitt auf, dessen Innenradius dem Außenradius des kugelsegmentförmigen Kopfes 10 entspricht. An den kugelsegmentförmigen Abschnitt schließt sich ein Halsabschnitt 15 an. Dieser hat die Form eines Segmentes eines Hohlkugelabschnittes und ist von dem kugelsegmentförmigen Abschnitt ausgehend nach außen divergent ausgebildet. Die Achse des Halsabschnittes geht durch den Mittelpunkt des hohlkugelsegmentförmigen Abschnittes. Auf der dem Halsabschnitt gegenüberliegenden Seite des kugelsegmentförmigen Abschnittes erstreckt sich senkrecht zu der Symmetrieachse von Halsabschnitt und kugelsegmentförmigen Abschnitt ein Aufnahmeschlitz 16, in dessen der anderen Kopfhälfte abgewandten Außenseite eine Versenkung 17 vorgesehen ist. Die Breite des Aufnahmeschlitzes 16 ist so gewählt, daß eine aufzunehmende Gewindestange lose durch diesen hindurchführbar ist. Auf diese Weise ist der Aufnahmeteil innerhalb eines Kegelwinkels um die Achse des Gewindeschaftteiles 7 frei schwenkbar und um die Achse der Schraube zum Ausrichten der Schlitzrichtung frei drehbar.

Zwischen dem Aufnahmeteil 8 und dem Gewindeteil 9 weist jede der Schrauben ein sich senkrecht zur Schraubenachse erstreckendes Zylinderstück 18-21 auf. An einem Ende des Zylinderstückes erstreckt sich senkrecht zur Zylinderachse eine Bohrung 23 durch dieses hindurch, deren Durchmesser so gewählt ist, daß ein Hals 22 der jeweiligen Schraube zwischen Kopf und Gewindeteil gerade in die Bohrung einsetzbar ist. Auf der anderen Seite des Zylinderstückes ist koaxial zu seiner Achse eine einen Bolzen 24 aufnehmende Bohrung 25 vorgesehen. Der Bolzen 24 weist senkrecht zu der Achse der Bohrung und damit zu der Zylinderachse eine Gewindebohrung auf. Die Abmessungen des Bolzens sind so gewählt, daß er in der Bohrung 25 frei um seine Achse drehbar ist. Senkrecht zu der Bohrung 23 und senkrecht zu der Bohrung 25 ist eine weitere Bohrung 26 in dem Zylinder angebracht. Diese ist koaxial zu der Gewindebohrung im Bolzen angeordnet, so daß eine Schraube durch diese hindurch in den Bolzen 24 einschraubbar ist. Die beiden Austrittsenden der Bohrung 26 weisen in einer Richtung parallel zur Schraubenachse kegelförmige Aufweitungen 27,28 auf, die ein Verschwenken der aufzunehmenden Schrauben innerhalb eines durch die Größe der kegelförmigen Aufweitung bestimmten Bereiches um die Längsachse des Zylinderstückes ermöglichen.

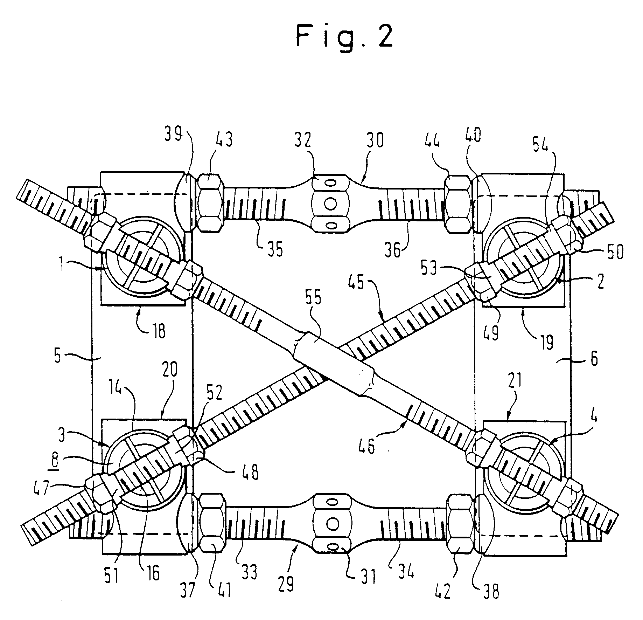

Die Korrektur- und Haltevorrichtung weist ferner ein Paar Gewindestangen 29,30 auf. Diese besitzen in einem zentralen Abschnitt einen koaxial ausgerichteten Sechskantteil 31,32 zum Ineingriffbringen mit einem Schraubschlüssel und beidseitig daran anschließende Gewindebereiche 33,34; 35,36. Die jeweiligen gegenüberliegenden Gewindebereiche 33,34 bzw. 35,36 einer Gewindestange weisen in ihrer Drehrichtung einander entgegengesetzte Rechts- bzw. Links-Gewinde auf. Für jeden Gewindeabschnitt ist ein quasi als Unterlegscheibe wirkendes Zwischenglied 37-40 vorgesehen, welches so ausgebildet ist, daß es auf seiner dem Sechskant zugewandten Seite eine ebene Fläche und auf seiner gegenüberliegenden Seite eine hohlzylindersegmentförmige Oberfläche aufweist. Letztere entspricht in ihrer Krümmung der Krümmung der Außenorberfläche der jeweiligen Zylinderstücke 18-21. Zwischen Sechskantteil und Zwischenglied ist jeweils eine Mutter 41-44 vorgesehen. Wie am besten aus Fig. 2 ersichtlich ist, ist eine Gewindestange 29 mit den beiden auf einer Seite der Wirbelsäule anzuordnenden Schrauben 3,4 über die verbindenden Zylinderstücke 20,21 durch Einschrauben der Gewindebereiche in die Bolzen 24 eingesetzt. Die Muttern 41-43 sind noch nicht angezogen, so daß eine ausreichende Bewegung gegeben ist und die Schrauben in die Wirbel einschraubbar sind. In entsprechender Weise ist die Gewindestange 30 über die zugehörigen Zylinderstücke mit den Schrauben 1 und 2 verbunden. Nach dem Einschrauben der Schrauben 1-4 in die zugehörigen Wirbelteile werden die Muttern 37-40 zunächst lose angezogen, so daß der Abstand der gegenüberliegenden Schrauben 1 und 2 und 3 und 4 fixiert ist.

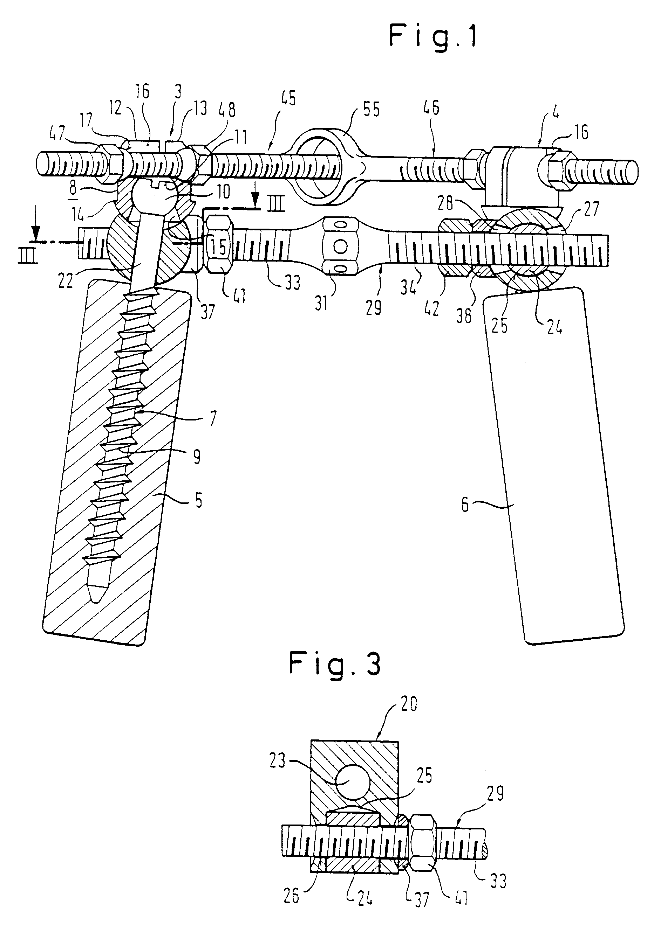

Ferner sind Gewindestangen 45 und 46 vorgesehen. Die Gewindestange 45 ist eine einfache Gewindestange mit zwei Mutternpaaren 47,48; 49,50. Die Mutternpaare weisen einander zugewandte und zur Gewindestange koaxial angeordnete kugelsegmentförmige Abschnitte 51,52 bzw. 53,54 auf. Die jeweiligen Kopfhälften des Aufnahmeteiles 8 weisen koaxial zu dem jeweiligen Aufnähmeschlitz 16 kugelsegmentförmige Versenkungen 17 auf, wie am besten aus Fig. 1 ersichtlich ist. Der Durchmesser der Gewindestange ist so gewählt, daß diese gerade in die Aufnähmeschlitze der Aufnahmeteile der Schrauben 1 und 4 einsetzbar ist. Die Gewindestange 46 unterscheidet sich von der beschriebenen Gewindestange 45 lediglich dadurch, daß sie in ihrem Mittenbereich eine Öse 55 bildenden Abschnitt aufweist. Ansonsten ist die Ausbildung entsprechend so gewählt, daß die Stange in den Aufnähmeschlitz der Schrauben 2 und 3 einsetzbar ist. Die Öse 55 dient zum Hindurchführen der Gewindestange 46 derart, daß die beiden Schrauben quasi in der gleichen Ebene mit den Schrauben 1,4 bzw. 2,3 verbindbar sind. Durch die Verschwenkbarkeit der Aufnahmeteile relativ zu den Schrauben ist das Einsetzen der Gewindestangen 45,46 auf einfache Weise möglich. Nach dem Einsetzen werden die jeweiligen Gewindeschraubenpaare zum vorläufigen Festlegen der Gewindestangen relativ zu den Aufnahmeteilen festgezogen.

Im Betrieb werden zunächst Löcher in das entsprechende Wirbelpaar 5,6 zur Aufnahme der Schrauben 1-4 gebohrt. Dann wird in Abhängigkeit von dem Abstand der den Schrauben 1 und 2 bzw. 3 und 4 entsprechenden Bohrung mit den Gewindestangen 29,30 ein entsprechender Abstand für die Schrauben 1 und 2 bzw. 3 und 4 eingestellt. Anschließend werden die Schrauben 1-4 in die vorbereiteten Löcher eingeschraubt. Im Anschluß daran werden die Gewindestangen 45 und 46 in die zugehörigen Schlitze eingesetzt. Durch Einstellen der Mutternpaare 47,48 bzw. 49,50 wird nun die Winkelstellung der Wirbel 5 und 6 zueinander einjustiert. Eine eventuelle Abstandsveränderung kann gleichzeitig bzw. im Wechselspiel über das Einstellen der Gewindestangen 29 und 30 erreicht werden. Im Anschluß an die gewünschte Ausrichtung werden die Muttern 41-44 und die Mutternpaare 47,48 und 49,50 festgezogen. Durch die oben beschriebene Ausbildung ist einerseits die genaue längenmäßige und winkelmäßige Ausrichtung der Wirbel möglich und kann auch zu einem späteren Zeitpunkt mit der gleichen Vorrichtung wiederholt werden. Gleichzeitig wird durch die sich kreuzenden Gewindestangen eine hohe Stabilität gegen Torsionskräfte erzielt. Im Hinblick darauf, daß auch nach der Ausrichtung keine Teile an der Vorrichtung entfernt werden, bleiben diese auch für zukünftige Änderungen der Einstellung voll funktionsfähig.

## Patentansprüche

1. Korrektur- und Haltevorrichtung, insbesondere für die Wirbelsäule, mit jeweils einen Gewindeschaftteil (7) und ein damit gelenkig verbundenes Aufnahmeteil (8) aufweisenden Paaren von Schrauben (1, 3; 2, 4), einem ersten Paar von Gewindestangen (45, 46) zum Verbinden von jeweils einer Stange mit zwei der Schrauben an einer ersten Verbindungsstelle an deren Aufnahmeteil (8) sowie einem zweiten Paar von Gewindestangen (29, 30), von denen jeweils eine mit zwei der Schrauben an einer gegenüber der ersten Verbindungsstelle zum Gewindeschaftteil (7) hin versetzten zweiten Verbindungsstelle zu verbinden ist.

2. Korrektur- und Haltevorrichtung nach Anspruch 1,
dadurch gekennzeichnet, daß Gewindeschaftteil (7) und Aufnahmeteil (8) der Schrauben (1-4) derart über ein Gelenk verbunden sind, daß eine Relativbewegung des Aufnahmeteiles (8) gegenüber dem Gewindeschaftteil (7) um eine erste Achse und um eine sich zur ersten Achse quer erstreckende zweite Achse möglich ist.

3. Korrektur- und Haltevorrichtung nach Anspruch 1 oder 2,
dadurch gekennzeichnet, daß die Stangen des zweiten Paares von Gewindestangen (29, 30) an ihren jeweiligen zur Verbindung mit den Schrauben dienenden Enden (33-36) entgegengesetzt laufende Gewinde aufweisen.

4. Korrektur- und Haltevorrichtung nach einem der Ansprüche 1 bis 3,
dadurch gekennzeichnet, daß das zweite Paar von Gewindestangen mit den auf jeweils einer Seite einer Wirbelsäule einzuschraubenden Schrauben (1, 3) verbindbar ist.

5. Korrektur- und Haltevorrichtung nach Anspruch 4,
dadurch gekennzeichnet, daß eine Stange (46) des ersten Paares eine Öse (55) zum Hindurchführen der zweiten Stange (46) des ersten Paares aufweist.

6. Korrektur- und Haltevorrichtung nach einem der Ansprüche 1 bis 5,
dadurch gekennzeichnet, daß jede Stange des ersten Paares der Gewindestangen (45, 46) einerseits mit der für die erste bzw. zweite Seite vorgesehenen Schraube (1, 3) des ersten Paares und andererseits mit der für die zweite bzw. erste Seite vorgesehenen Schraube (4, 2) des zweiten Paares verbindbar ist.

## Claims

1. A correction and restraining devise, in particular for the vertebral column, with pairs of screws (1, 3; 2, 4) each having one threaded shank part (7) and one receiving part (8) articulated thereto, a first pair of threaded pins (45, 46) for connecting one pin respectively to two of the screws at a first connecting point on its receiving part (8), as well as a second pair of threaded pins (29, 30), one of which is to be respectively connected to two of the screws at a second connecting point that is offset relative to the first connecting point towards the threaded shank part (7).

2. A correction and restraining device according to claim 1, characterized in that the threaded shank part (7) and receiving part (8) of the screws (1-4) are connected via a joint in such a way that a relative movement of the receiving part (8) relative to the threaded shank part (7) round a first axis and round a second axis extending transversely to the first axis is possible.

3. A correction and restraining device according to claim 1 or 2, characterized in that the pins of the second pair of threaded pins (29, 30) have at their respective ends (33, 36) serving for the connection to the screws, threads running in opposite directions.

4. A correction and restraining device according to one of claims 1 to 3, characterized in that the second pair of threaded pins can be connected to the screws (1, 3) to be screwed in on one side each of a vertebral column.

5. A correction and restraining device according to claim 4, characterized in that one pin (46) of the first pair has an eyelet (55) for the second pin (46) of the first pair to pass through.

6. A correction and restraining device according to one of claims 1 to 5, characterized in that each pin of the first pair of the threaded pins (45, 46) can be connected on the one hand to the screw (1, 3) of the first pair provided for the first or second side respectively and on the other hand, to the screw (4, 2) of the second pair provided for the second or first side respectively.

## Revendications

1. Dispositif de maintien et de correction, notamment de la colonne vertébrale, comportant des paires de vis (1, 3 ; 2,4) dont chacune présente une partie broche filetée (7) et, articulée sur celle-ci, une partie réception (8), et une première paire de tiges filetées (45, 46) dont chacune doit être assemblée à deux des vis, en un premier point d'assemblage situé sur leur partie réception (8), ainsi qu'une deuxième paire de tiges filetées (29, 30) dont chacune doit être assemblée à deux des vis, en un deuxième point d'assemblage lequel, par rapport au premier, est décalé en direction de la partie broche filetée (7).

2. Dispositif de maintien et de correction selon la revendication 1, caractérisé en ce que la partie broche filetée (7) et la partie réception (8) des vis (1 à 4) sont reliées par une articulation de manière à permettre un mouvement de la partie réception (8) par rapport à la partie broche filetée (7) d'une part, autour d'un premier axe et, d'autre part, autour d'un deuxième axe s'étendant transversalement au premier.

3. Dispositif de maintien et de correction selon la revendication 1 ou 2, caractérisé en ce que les tiges de la deuxième paire de tiges filetées (29, 30) présentent, à leurs extrémité (33, 36) qui servent à les assembler aux vis, des filetages dont les sens sont opposés.

4. Dispositif de maintien et de correction selon l'une des revendications 1 à 3, caractérisé en ce que la deuxième paire de tiges filetées peut être assemblée aux vis (1, 3), dont chacune doit être vissée sur un côté de la colonne vertébrale.

5. Dispositif de maintien et de correction selon la revendication 4, caractérisé en ce qu'une tige (46) de la première paire présente un oeillet (55) pour le passage de la deuxième tige (46) de ladite première paire.

6. Dispositif de maintien et de correction selon l'une des revendications 1 à 5, caractérisé en ce que chaque tige de la première paire de tiges filetées (45, 46) peut être assemblée d'une part, à la vis (1, 3) de la première paire prévue pour le premier (ou le deuxième) côté et, d'autre part, à la vis (4, 2) de la deuxième paire prévue pour le deuxième (ou le premier) côté.
